Europäisches Patentamt

European Patent Office

Office européen des brevets

(11) Publication number: **0 261 862 B1**

# EUROPEAN PATENT SPECIFICATION

(45) Date of publication of patent specification: **12.05.93**  (51) Int. Cl.5: **C12N 5/04**, //C12P19/44, C12P7/24

(21) Application number: **87308155.8**

(22) Date of filing: **15.09.87**

(54) **Process for culturing saffron stigma tissues.**

(30) Priority: **20.09.86 JP 222500/86**
**30.05.87 JP 137440/87**

(43) Date of publication of application:
**30.03.88 Bulletin 88/13**

(45) Publication of the grant of the patent:
**12.05.93 Bulletin 93/19**

(84) Designated Contracting States:
**DE ES FR GB IT**

(56) References cited:
**EP-A- 0 052 001**
**EP-A- 0 233 040**

**I.K. VASIL: "Cell culture and somatic cell genetics of plants", vol. 1, 1984, chapter 27, Academic Press, Inc., Orlando, US; T.S. RANGAN: "Culture of ovaries"**

**I.K. VASIL: "Cell culture and somatic cell genetics of plants", vol. 1, 1984, chapter 3, Academic Press, Inc., Orlando, US; O.L. GAMBORG: "Plant cell cultures: nutrition and media"**

(73) Proprietor: **OHTA's ISAN CO., LTD**
**2-3, 2-chome, Sengoku Bunkyo-ku Tokyo(JP)**

(72) Inventor: **Kohda, Hiroshi**
**No. 102, 3-3-3 Ushidawaseda**
**Higashi-ku Hiroshima-shi(JP)**
Inventor: **Koyama, Atsuko No. 303, Mitsui Ogatayamashataku C-5 1-2 Misono Otake-shi Hiroshima-shi(JP)**
Inventor: **Fujioka, Naomi**
**5-25, 5-chome Nagatsuka Asaminami-ku Hiroshima-shi(JP)**
Inventor: **Ohta, Yoshiaki**
**21-4, 1-chome Minamimagome Ohta-ku Tokyo(JP)**
Inventor: **Hosono, Tsuyoshi**
**No. 8-403, 17, 4-chome Isobe Chiba-shi,Chiba-ken(JP)**
Inventor: **Yamasaki, Kazuo 8-2, 1-come Misuzugaoka-Higashi Saeki-ku Hiroshima-shi(JP)**
Inventor: **Miyagawa, Hideki**
**1o-9 Misasakita-machi Nishi-ku Hiroshima-shi(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

AGRICULTURAL AND BIOLOGICAL CHEMIS-
TRY, vol. 51, no. 9, September 1987, pages
2395–2400, Tokyo, JP; H. HIMENO et al.:
"Synthesis of crocin, picrocrocin and
safranal by saffron stigma–like structures
proliferated in Vitro"

Inventor: **Omori, Yuki No. 303 Corpo Shibao
11–10 3–chome Nagahama–machi
Oita–shi Oita–ken(JP)**
Inventor: **Itoh, Hiroshi
12–13, 3–chome Mama
Ichikawa–shi Chiba–ken(JP)**

(74) Representative: **Pearce, Anthony Richmond et
al
MARKS & CLERK Alpha Tower Suffolk Street
Queensway Birmingham B1 1TT (GB)**

## Description

This invention relates to a process for producing a substance having useful components of saffron such as crocein and the like on a large scale by tissue culture of the pistils of saffron (Crocus sativus Linne), which is an iridaceous plant.

The stigma moiety of saffron contains carotenoid pigments such as crocein and the like, picrocrocein which is a bitter glycoside, and saffronal which is an odoriferous element, and thus has been widely used for medicinal purposes and also as color no agents and spices of food.

At present, the amount of its domestic consumption in Japan increases steadily each year, amounting to about 1 ton.

The dried pistils of saffron are extremely expensive, costing as much as from 300 thousand yen to a million yen per kg.

Conventional Technologies

As a process for producing a dried product of the stigmata of saffron, a method of cultivating saffron in a field, picking the stigmata within the term of the flowering time and drying them has conventionally been employed, but no producing method using a process of tissue culture of saffron stigma−like tissues is known to us apart from a method described in EP−A−0233040 published on 19 August 1987 in respect of the contracting States, Spain, France and Italy.

Problems to be Solved by the Invention

The conventional method of cultivating saffron in a field has the following disadvantages:

a. Saffron has an inconvenience in which only 3.6 kg − 5.4 kg (as dried saffron) per acre can be obtained even if the usual conditions are complete, so that the yield is low.

b. The flowering time is from October to November, during which the flowers must be picked. As the flowering time is as short as 15 days, however, unpicked flowers remain if picking is not started simultaneously with the blooming.

c. The growth of saffron is influenced by the natural environment and weather, so that an unseasonable weather inconveniently results in an extreme reduction of the the yield.

d. Saffron is mainly cultivated in European countires such as Spain and France and in Asia Minor, and also cultivated in Japanese territory, expecially in the Kyushu district and the Chugoku district.

As repeated cultivation often causes blight, however, the cultivation cannot be repeated for 5 years or more.

Thus, an increase in yield is hopeless in these circumstances.

EP−A−0233040 referred to above discloses a method for the production of a stigma of Crocus sativus L. and the corresponding organs in vitro, wherein at least part of the gynoecium containing the stigma and/or the ovary is transplanted and cultured in single type of culture medium (eg LS or B5 medium) containing both a cytokinin (eg benzyl/amino purine kinetin or zeatin) and an auxin (eg IAA,NAA or IBA), or only a cytokinin. The successive culturing in LS medium containing 10 mg/l of NAA and 1 mg/l of kinetin, and then in LS medium containing no NAA but 5 mg/l of kinetin, followed by further culturing in LS medium containing 5 mg/l of kinetin and 0.1 mg/l of NAA is disclosed.

In view of the previously described disadvantages of the conventional cultivation method, the present inventor has invented a process for culturing stigma tissues of saffron which can provide its mass production at any time in any place without being influenced by the harvesting season.

For the Contracting States DE GB

According to the present invention, there is provided a process for culturing saffron stigma tissues which comprises the following steps:

(a) picking the portion containing at least the upper, middle and lower parts (o, p and q) of the moiety continued from petal to ovary of saffron and cutting said portion into parts,

(b) transplanting the cut parts on a liquid or solid LS medium or B5 medium, said medium containing at least one cytokinin selected from benzylaminopurine, kinetin and zeatin and at least one auxin selected from NAA, IBA and IAA as main hormones, and

(c) subculturing the transplanted parts under stationary, rotary or shaking culture.

3

Preferably, the process further includes the step of:

transplanting the parts cultured on the LS medium or B5 medium to the B5 or LS medium respectively, followed by subculturing.

For the Contracting States ES FR IT

According to the present invention, there is provided a process for culturing saffron stigma tissues which comprises the following steps:

(a) picking the portion containing at least the upper, middle and lower parts (o, p and q) of the moiety continued from petal to ovary of saffron and cutting said portion into parts,

(b) transplanting the cut parts on a liquid or solid LS medium or B5 medium, said medium containing at least one cytokinin selected from benzylaminopurine, kinetin and zeatin and at least one auxin selected from NAA, IBA, IAA as main hormones,

(c) subculturing the transplanted parts under stationary, rotary or shaking culture, and

(d) transplanting the parts subcultured on the LS medium or B5 medium to the B5 or LS medium respectively, followed by subculturing, and in which the concentration of the main hormones in the medium for successive transplantation is lower than in the previous medium.

Benzylaminopurine (BAP) is also called $6 - $ benzyladenine (BA) NAA is $1 - $ naphthalenic acid, IBA is indolebutyric acid and IAA is indole $-3-$ acetic acid.

As further hormones which can be used with the main hormone, auxins such as $2,4 -$ dichlorophenoxy acetic acid $(2,4 - D)$, $4 -$ chlorophenoxy acetic acid (CPA) and the like, and growth hormones such as gibberellic acid $(GA_3)$, abscisic acid (ABA) and the like may be used.

The inorganic component in the medium include inorganic salts containing elements such as nitrogen, phosphorus, potassium, calcium, magnesium, sulfur, iron, manganese, zinc, boron, molybdenum, chlorine, iodine, cobalt and the like.

Examples of the carbon source in the medium include hydrocarbons such as sucrose and derivative thereof, organic acids such as fatty acid, and primary alcohols such as ethanol.

Vitamins in the medium include biotin, thamine (vitamin $B_1$), pyridoxine (vitamin $B_6$), pyridoxol, pyridoxamine, calcium pantothenate, ascorbic acid (vitamin C), inositol, nicotinic acid, nicotinic amide, riboflavin (vitamin $B_2$) and the like.

As amino acids in the medium, for example, glycine, alanine, glutamic acid, cysteine, tyrosine, aspartic acid, amide arginine, and lysine may be used.

This invention is further illustrated in more detail according to the concrete embodiments.

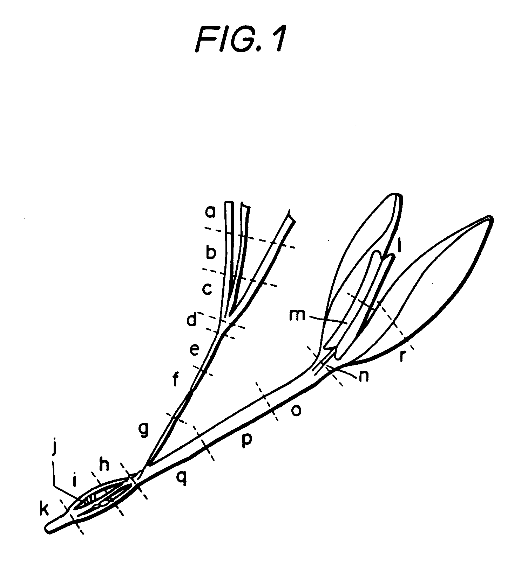

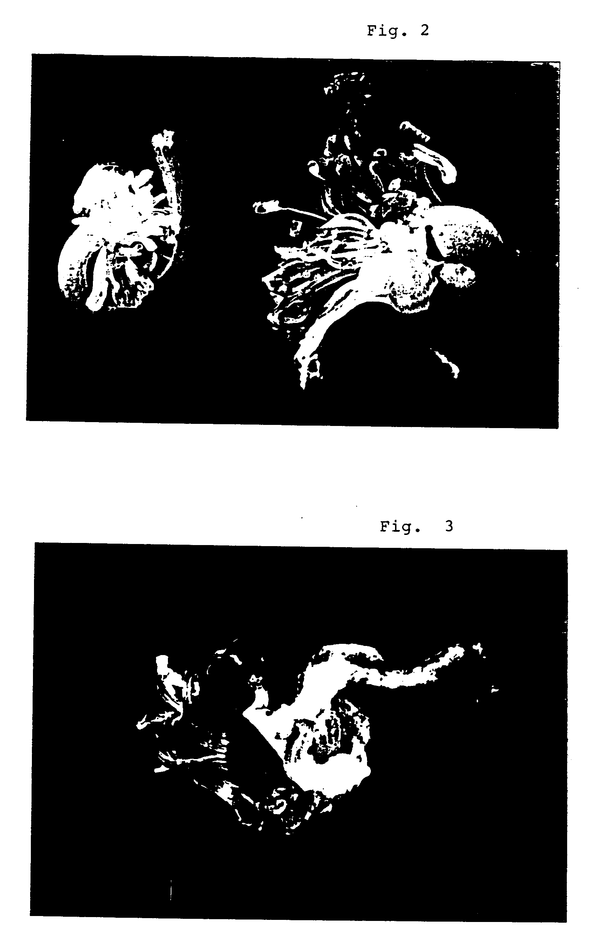

Figure 1 is a perspective view illustrating each moiety of the flower of saffron, and Figure 2 is an enlarged photograph of the tissue q cultured in No. 25 of B5 medium (containing BA and NAA)

| a | Upper part of stigma |
|---|---|
| b | Middle part of stigma |
| c | Lower part of stigma |
| d | Stigma style |
| e | Upper part of style |
| f | Middle part of style |
| g | Lower part of style |
| h | Upper part of ovary |
| i | Lower part of ovary |
| j | Ovule |
| k | Part under ovule |
| l | Upper part of anther |
| m | Lower part of anther |
| n | Filament |
| r | Petal |
| o | Upper part of the moiety continue from petal to ovary |
| p | Middle part of the moiety continue from petal to ovary |
| q | Lower part of the moiety continued from petal to ovary |

Example 1

Under the conditions as shown below, experiments over several generations were conducted, with respect to various concentration of each hormone and combination thereof, each medium and each culturing method, to find out the condition in which a tissue containing a yellow pigment can be cultured.

### a. Preparation of section

A bud grown to about 6 − 13 cm from the bulb is cut off from the base and washed in flowing water.

The washed bud is dipped in an Osvan (Trade Mark of Takeda Chemical Industries) 100 − fold solution for 5 minutes, in a Purelux (Trade Mark of Kabushiki Kaisha Ohyalux) 10 − fold solution for 5 minutes, and in 70% ethyl alcohol for 2 − 3 seconds, and then washed with sterilized water three times. After washing, the bud is taken out under an aseptic condition in a laboratory dish, and the stigma is cut off at the position slightly lower than the trilobate part, and the portion from the stigma to the ovary upper part is cut into 2 to 4 pieces so as to have a length of about 1 − 2.5 cm, for preparing planting sections.

### b. Preparation of medium

As the basic medium, a liquid or 0.2 % Gellan gum − containing solid medium of Linsmaier Skoog medium (sucrose 30 g/l, pH 5.7 − 5.8) (hereinafter referred to as LS medium), Ganborg B5 medium (sucrose 20 g/l, pH 5.7 − 5.8) (hereinafter referred to as 85 medium), and White medium (sucrose 20 g/l, pH 5.7 − 5.8) were used.

The pH was adjusted using 0.1N KOH and 0.1N HCl after addition of the hormones.

### c. Culturing method

(1) Stationary culture

Stationary culture was carried out in a dark place with keeping the culturing room at 25±3°C.

(2) Rotary culture

Rotary culture was carried out under the condition of 2 rpm (Kitagawa Tekkosho KW − 1) in darkness with keeping the culturing room at 22±1°C.

(3) Shaking culture

Shaking culture was carried out under the condition of 120 rpm (TAIYO ROTARY SHAKER R − 11) in darkness with keeping the culturing room at 22±1°C.

### d. Hormones

The added hormones are selected from Ba [6 − benzyl − adenine, also referred to as BAP (6 − benzylaminopurine)], zeatin, or kinetin (Ki) as the cytokinin and from IAA (indoleacetic acid), NAA (naphthaleneacetic acid), IBA (indolebutyric acid) or 2,4 − D (2,4 − dichlorophenoxyacetic acid) as the auxin.

The subculture was conducted by adding the combination of cytokinin and auxin as shown in Tables 1, 2, and 3 to each medium.

Table 1    B5 Medium    Unit: mol/l

| Auxin \ BA | 0 | $10^{-7}$ | $10^{-6}$ | $10^{-5}$ | $3 \times 10^{-5}$ |
|---|---|---|---|---|---|
| 0 | 1 | 6 | 11 | 16 | 21 |
| $10^{-7}$ | 2 | 7 | 12 | 17 | 22 |
| $10^{-6}$ | 3 | 8 | 13 | 18 | 23 |
| $10^{-5}$ | 4 | 9 | 14 | 19 | 24 |
| $5 \times 10^{-5}$ | 5 | 10 | 15 | 20 | 25 |

Table 2    LS Medium    Unit: mol/l

| Auxin \ BA | 0 | $10^{-7}$ | $10^{-6}$ | $10^{-5}$ | $3 \times 10^{-5}$ |
|---|---|---|---|---|---|
| 0 | 1' | 6' | 11' | 16' | 21' |
| $10^{-7}$ | 2' | 7' | 12' | 17' | 22' |
| $10^{-6}$ | 3' | 8' | 13' | 18' | 23' |
| $10^{-5}$ | 4' | 9' | 14' | 19' | 24' |
| $5 \times 10^{-5}$ | 5' | 10' | 15' | 20' | 25' |

Table 3    White Medium    Unit: mol/l

| Auxin \ BA | 0 | $10^{-7}$ | $10^{-6}$ | $10^{-5}$ | $3 \times 10^{-5}$ |
|---|---|---|---|---|---|
| 0 | 1" | 6" | 11" | 16" | 21" |
| $10^{-7}$ | 2" | 7" | 12" | 17" | 22" |
| $10^{-6}$ | 3" | 8" | 13" | 18" | 23" |
| $10^{-5}$ | 4" | 9" | 14" | 19" | 24" |
| $5 \times 10^{-5}$ | 5" | 10" | 15" | 20" | 25" |

(Figures in the tables indicate medium numbers)

The subculture was conducted every time by transplanting the tissues having r = 5 − 10 mm (r represents the diameter of tissue) successively at intervals of 5 − 8 weeks.

e. Results

The state of the tissue or callus of the 4th generation was confirmed, and the results as shown in the following Tables 4 − 6 were obtained.

6

Table 4

| B5 Medium (Auxin: NAA) | |
| --- | --- |
| State of tissue or callus | Observed medium number |
| White to yellow callus was derived in 7 weeks | 15, 19, 20, 24, 25 |
| White to orange stigma − like tissues of 5 − 6 mm were differentiated in 3 months | 15, 19, 20, 24, 25 |
| Yellow to orange stigma − like tissues were differentiated in 4 months | 15, 19, 20, 24, 25 |

Particularly, in Nos. 24 and 25 mediums, a great number of stigma − like small process tissues were observed.

Table 5

| LS Medium (Auxin: NAA) | |
| --- | --- |
| State of tissue or callus | Obserbed medium number |
| White, transparent and soft callus was differentiated (partly hard) | 9', 10', 14', 15' 19', 20', 24', 25' |
| White stigma − like tissues were differentiated in 7 weeks to 3 months | 15', 19', 20', 24' 25' |
| A number of orange to yellow stigma − like tissues were differentiated in 3 − 4 months | 15', 19', 20', 24' 25' |

Table 6

| White Medium (Auxin: NAA) | |
| --- | --- |
| States of tissue or callus | Observed medium number |
| White to yellow callus was derived in 7 weeks | 19'', 20'', 24'' |

The tissues in Nos. 15, 19, 20, 24, and 25 of B5 mediums in which a great number of stigma − like small process tissues were observed were transplanted to Nos. 7', 8', 9', 12', 13', and 14' of LS mediums, and the result shown in Table 7 was obtained.

Also, those in Nos 15', 19', 20', 24', and 25' of LS mediums in which a great number of stigma − like small process tissues were observed were transplanted to Nos. 7, 8, 9, 12, 13, and 14 of B5 mediums, and the result shown in Table 8 was obtained.

Table 7

| Each No. of B5 medium ⇒ LS medium | |
| --- | --- |
| States of tissue or callus | Observed medium number |
| Changed to stigma − like tissues | All numbers |
| Trilobate tissues similar to the original plant were observed | 14' |

The tissues in Nos. 7' − 9' have a tendency to become deeper red than those in Nos. 12' − 14'.

Table 8

| Each No. of LS medium ⇒ B5 medium | |
| --- | --- |
| States of tissue or callus | Observed medium number |
| Changed to stigma − like tissues<br>Trilobate tissues similar to the original plant were observed | All numbers<br>14 |

The tissues in Nos. 7 − 9 have a tendency to become deeper red than those in Nos. 12 − 14.

From the above results, it was proved that as the medium, LS mediums and B5 mediums are excellent for tissue culture (tissues having yellow pigments can be cultured) and derivation of callus, excepting a number of White mediums.

As to the culturing method, all of stationary, rotary and shaking cultures were found to be suitable.

With respect to the combination in the medium numbers which afforded a satisfactory result among the LS mediums and B5 mediums containing NAA as the auxin and BA as the cytokinin, zeatin or kinetin was used instead of BA and almost the same result as the case of BA was obtained.

Also, when IBA, 2,4 − D or IAA was used instead of NAA, mediums capable of culturing were hardly found for 2,4 − D, and the tissue culture could be performed for IBA and IAA, although inferior compared with the case of NAA.

This shows that as the added hormones, BA is preferred as the cytokinin and NAA as the auxin.

Examination of the relation of the addition amounts of BA and NAA confirmed that Nos. 15, 19, 20, 24, and 25 of B5 medium in Table 1, Nos. 15', 19', 20', 24', and 25' of LS medium in Table 2, and Nos. 19'', 20'' and 24'' of White medium in Table 3 are excellent for tissue culture.

This shows that the hormone amounts of $10^{-6}$ − $3 \times 10^{-5}$ for BA and $10^{-5}$ − $5 \times 10^{-5}$ for NAA (unit is mol/l) suitable in B5 medium and LS medium, and the hormone amounts of $10^{-6}$ − $3 \times 10^{-5}$ for BA and $10^{-5}$ − $5 \times 10^{-5}$ for MAA (unit is mol/l) are suitable in White medium.

The subculture need not be conducted in the same medium, and the similar result can be obtained in transplanting tissues from B5 medium to LS medium or vice versa. In the case of successive transplan − tation, however, the culture is desirably conducted at a hormone concentration lower than that of the previous medium.

Further, it was confirmed that those in which the tissues cultured in Nos. 15, 19, 20, 24 and 25 of B5 medium are transplanted to Nos. 7', 8', 9', 12', 13', and 14' of LS medium containing BA and NAA, those in which the tissues cultured in Nos. 15', 19', 20', 24', and 25' of LS medium are transplanted to Nos. 7, 8, 9, 12, 13, and 14 of LS medium containing BA and NAA, and those using the LS medium and B5 medium containing BA of $10^{-7}$ and IAA of $10^{-7}$ M, are excellent for a high pigment − productive culturing.

As the B5 medium, Nos. 15, 19, 20, 24, and 25 containing BA and NAA proved to be excellent.

This shows that the hormone amounts in B5 medium and LS medium are suitably $10^{-7}$ − $10^{-6}$ for BA and $10^{-7}$ − $10^{-5}$ for NAA (mol/l) for high production of pigments.

As a result of TLC analysis and HPLC analysis, the cultured tissues proved to contain crocein and picrocrocein, similarly to the original plant.

Example 2

Under the conditions as shown below, experiments over several generations were conducted, with respect to various concentration of each hormone and combination thereof, each medium and each culturing method, to find out the condition in which a tissue containing a yellow pigment can be cultured.

a. Preparation of section

A bud grown to about 6 − 13 cm from the bulb is cut from the base and washed with flowing water.

The washed bud is dipped in an Osvan 100 − fold solution for 5 minutes, a Purelux 10 − fold solution for 5 minutes, and 70 % ethyl alcohol for 2 − 3 seconds, and then washed with sterilized water three times. After washing, the bud is taken out under an aseptic condition in a laboratory dish and cut into the portions a − r as shown in the figure to prepare planting sections.

8

# EP 0 261 862 B1

### b. Preparation of medium

As the basic medium, fixed mediums in which 0.9 % agar or 0.2 % Gelrite (a Trade Mark of Kelco Division of Merck & Co) was added to Linsmaier Skoog (LS) medium (Sucrose 30 g/l, pH 5.7 − 5.8). Ganborg B5 medium (sucrose 20 g/l, pH 5.7 − 5.8) and White medium (sucrose 20 g/l, pH 5.7 − 8) were prepared.

The pH was adjusted using 0.1N KOH and 0.1N HCl after addition of the hormones.

### c. Culturing method

Stationary, rotary, or shaking culture was carried out in a dark place with keeping the culturing room at 25±3°C.

### d. Plant hormone

As the plant hormones, a cytokinin and an auxin were added under various concentrations as shown in Table 9.

Each hormone used in the experiment is as follows:

Cytokinin:   6 − benzyladenine (BA), kinetin (Ki), zeatin

Auxin:   indole − 3 − acetic acid (IAA), 2,4 − dichlorophenoxyacetic acid (2,4 − D), 1 − naphthalenic acid (NAA), indolebutyric acid (IBA)

**Table 9**    Unit: mol/l

| Cytokinin / Auxin | 0 | $10^{-7}$ | $10^{-6}$ | $10^{-5}$ | $3 \times 10^{-5}$ |
|---|---|---|---|---|---|
| 0 | 1 | 6 | 11 | 16 | 21 |
| $10^{-7}$ | 2 | 7 | 12 | 17 | 22 |
| $10^{-6}$ | 3 | 8 | 13 | 18 | 23 |
| $10^{-5}$ | 4 | 9 | 14 | 19 | 24 |
| $5 \times 10^{-5}$ | 5 | 10 | 15 | 20 | 25 |

**(Figures in the table indicate number of medium)**

The subculture was conducted every time by transplanting the tissues having r = 5 − 10 mm (r represents the diameter of tissue) successively at intervals of 5 − 8 weeks.

### e. Results

As a result of examining the state of the tissues of the 4th generation, emergence of stigma − like tissues was observed in both B5 medium and LS medium, as shown in the following Tables 10 − 12.

9

Table 10

| Part | B5 Medium, LS Medium | | | | | | | | | | | | | | |
|---|---|---|---|---|---|---|---|---|---|---|---|---|---|---|
| | Medium No. of BA − NAA combination | | | | | | | | | | | | | | |
| | 11 | 12 | 13 | 14 | 15 | 16 | 17 | 18 | 19 | 20 | 21 | 22 | 23 | 24 | 25 |
| a | X | X | X | X | X | X | X | X | X | X | X | X | X | X | X |
| b | X | X | X | X | X | X | X | X | X | X | X | X | X | X | X |
| c | X | X | △ | △ | △ | X | X | △ | △ | △ | X | X | △ | △ | △ |
| d | X | X | △ | △ | ○ | X | X | △ | ○ | ○ | X | X | ○ | ○ | ○ |
| e | X | X | △ | △ | ○ | X | X | △ | ○ | ○ | X | X | ○ | ○ | ○ |
| f | X | X | △ | △ | ○ | X | X | △ | ○ | ○ | X | X | ○ | ○ | ○ |
| g | X | X | △ | △ | ○ | X | X | △ | ○ | ○ | X | X | ○ | ○ | ○ |
| h | X | X | △ | △ | ◎ | X | X | ○ | ○ | ◎ | X | X | ○ | ◎ | ◎ |
| i | X | X | △ | △ | ○ | X | X | △ | ○ | ○ | X | X | ○ | ○ | ○ |
| j | X | X | △ | △ | △ | X | X | △ | △ | △ | X | X | △ | △ | △ |
| k | X | X | △ | △ | ○ | X | X | △ | ○ | ○ | X | X | ○ | ○ | ○ |
| ℓ | X | X | X | X | X | X | X | X | X | X | X | X | X | X | X |
| m | X | X | X | X | X | X | X | X | X | X | X | X | X | X | X |
| n | X | X | X | X | X | X | X | X | X | X | X | X | X | X | X |
| o | X | X | △ | △ | ◎ | X | X | ○ | ○ | ◎ | X | X | ○ | ◎ | ◎ |
| p | X | X | △ | △ | ◎ | X | X | ○ | ◎ | ◎ | X | X | ○ | ◎ | ◎ |
| q | X | X | △ | △ | ◎ | X | X | ○ | ◎ | ◎ | X | X | ○ | ◎ | ◎ |
| r | X | X | X | X | X | X | X | X | X | X | X | X | X | X | X |

◎, ○, △, and X indicate the number of emergences of stigma − like tissues per piece after culturing for 4 months; ◎: 31 or more, ○: 16 − 30, △: 5 − 15, X: hardly emerged.

EP 0 261 862 B1

Table 11

| Part | Medium No. of BA – IAA combination | | | | | | | | | | | | | | |
|------|----|----|----|----|----|----|----|----|----|----|----|----|----|----|----|
| **B5 Medium, LS Medium** | | | | | | | | | | | | | | | |
|      | 11 | 12 | 13 | 14 | 15 | 16 | 17 | 18 | 19 | 20 | 21 | 22 | 23 | 24 | 25 |
| a | X | X | X | X | X | X | X | X | X | X | X | X | X | X | X |
| b | X | X | X | X | X | X | X | X | X | X | X | X | X | X | X |
| c | X | X | X | X | △ | X | X | X | △ | △ | X | X | △ | △ | △ |
| d | X | X | X | X | △ | X | X | X | △ | ○ | X | X | △ | ○ | ○ |
| e | X | X | X | X | △ | X | X | X | △ | ○ | X | X | △ | ○ | ○ |
| f | X | X | X | X | △ | X | X | X | △ | ○ | X | X | △ | ○ | ○ |
| g | X | X | X | X | △ | X | X | X | △ | ○ | X | X | △ | ○ | ○ |
| h | X | X | X | X | △ | X | X | X | △ | ○ | X | X | ○ | ○ | ○ |
| i | X | X | X | X | △ | X | X | X | △ | ○ | X | X | △ | ○ | ○ |
| j | X | X | X | X | △ | X | X | X | △ | △ | X | X | △ | △ | △ |
| k | X | X | X | X | △ | X | X | X | △ | ○ | X | X | △ | ○ | ○ |
| ℓ | X | X | X | X | X | X | X | X | X | X | X | X | X | X | X |
| m | X | X | X | X | X | X | X | X | X | X | X | X | X | X | X |
| n | X | X | X | X | X | X | X | X | X | X | X | X | X | X | X |
| o | X | X | X | X | △ | X | X | X | △ | ○ | X | X | ○ | ○ | ○ |
| p | X | X | X | X | △ | X | X | X | ○ | ○ | X | X | ○ | ○ | ○ |
| q | X | X | X | X | △ | X | X | X | ○ | ○ | X | X | ○ | ○ | ○ |
| r | X | X | X | X | X | X | X | X | X | X | X | X | X | X | X |

◎, ○, △, and X inducate the number of emergences of stigma – like tissues per piece after culturing for 4 months; ◎: 31 or more, ○: 16 – 30, △: 5 – 15, and X: hardly emerged.

11

Table 12

| | B5 Medium, LS Medium | | | | | | | | | | | | | | |
|---|---|---|---|---|---|---|---|---|---|---|---|---|---|---|---|
| Part | Medium No. of BA – IBA combination | | | | | | | | | | | | | | |
| | 11 | 12 | 13 | 14 | 15 | 16 | 17 | 18 | 19 | 20 | 21 | 22 | 23 | 24 | 25 |
| a | X | X | X | X | X | X | X | X | X | X | X | X | X | X | X |
| b | X | X | X | X | X | X | X | X | X | X | X | X | X | X | X |
| c | X | X | X | X | △ | X | X | X | △ | △ | X | X | △ | △ | △ |
| d | X | X | X | X | △ | X | X | X | △ | O | X | X | △ | O | O |
| e | X | X | X | X | △ | X | X | X | △ | O | X | X | △ | O | O |
| f | X | X | X | X | △ | X | X | X | △ | O | X | X | △ | O | O |
| g | X | X | X | X | △ | X | X | X | △ | O | X | X | △ | O | O |
| h | X | X | X | X | △ | X | X | X | △ | O | X | X | O | O | O |
| i | X | X | X | X | △ | X | X | X | △ | O | X | X | △ | O | O |
| j | X | X | X | X | △ | X | X | X | △ | △ | X | X | △ | △ | △ |
| k | X | X | X | X | △ | X | X | X | △ | O | X | X | △ | O | O |
| ℓ | X | X | X | X | X | X | X | X | X | X | X | X | X | X | X |
| m | X | X | X | X | X | X | X | X | X | X | X | X | X | X | X |
| n | X | X | X | X | X | X | X | X | X | X | X | X | X | X | X |
| o | X | X | X | X | △ | X | X | X | △ | O | X | X | O | O | O |
| p | X | X | X | X | △ | X | X | X | O | O | X | X | O | O | O |
| q | X | X | X | X | △ | X | X | X | O | O | X | X | O | O | O |
| r | X | X | X | X | X | X | X | X | X | X | X | X | X | X | X |

◎, ○, △, and X indicate the number of emergences of stigma – like tissues per piece after culturing 4 months; ◎: 31 or more, ○: 16 – 30, △: 5 – 15, and X: hardly emerged.

In Nos. 1 – 10 mediums having each combination of hormones, stigma – like tissues did not emerge.

The state of the tissues in No. 25 shown in Table 10 was obtained by growing as shown in Table 1 for B5 medium and in Table 2 for LS medium after 3 months.

① In the White mediums, no emergence was observed. This suggests that White medium is not suitable for culturing stigma – like tissues of saffron.

In a comparison of B5 medium with LS medium, the number of emergences was higher in B5 medium by about 10 %.

② The mediums having the combination of BA and 2,4 – D showed no emergence in any medium number. This suggests that the auxin 2,4 – D is not suitable for culturing stigma – like tissues.

Next, as a result of examining preferable auxins, NAA, IA and IBA were preferred in the order named.

③ The planting sections a, b, l, m, and n did not show the emergence of stigma – like tissues under any culturing conditions.

With respect to the emerging state of stigma – like tissues, it was found that q, p, o, h, d, e, g and f are excellent in the order named, and q, p, and o are preferable, and so at least these parts are used in the present invention ④ Further, as to the mediums in which the results of mark ○ or ◎ are obtained in each number of BA – NAA, BA – IAA, and BA – IBA, when Ki or zeatin was added instead of BA, almost the same result was obtained.

For the growing degree (the degree of growing the tissue), BA proved to be better than Ki by 5 % or so.

12

Accordingly, BA is preferable as the cytokinin.

However, there were hardly differences along the cytokinins. The reason can be considered that all the cytokinins belong to adenin.

⑤ When preferable concentrations of the cytokinin and the auxin were examined, it was found that, as a whole, the concentrations of $10^{-6} - 3 \times 10^{-5}$ mol/l for the cytokinin and $10^{-6} - 5 \times 10^{-5}$ mol/l for the auxin are preferable, although varied depending on planting sections.

⑥ Although agar and Gelrite were used to make a solid medium, the growing degree is 5 − 10% higher in those containing 0.2% Gelrite than those containing 0.9 % agar.

In this example, the mediums having the combinations indicated in Nos. 1 − 12 did not show the emergence of stigma tissues, but when the tissues emerged in Nos. 24 or 25 were transplanted successively to Nos. 7, 8, 9, 10, 12, 13, 14, or 15 similarly to the first example described above, the stigma tissues were cultured to produce the pigments more highly.

This revealed that the culture is preferably conducted at low concentrations of the hormones, after the tissues have been once emerged.

This effect was further increased by adding $10^{-6} - 10^{-5}$ M of gibberellic acid (GA$_3$).

Effects

As illustrated so far, according to the process for tissue culture of this invention, saffron stigma tissues can be produced in a large scale and in a short time without limiting the harvesting season and the districts, compared with the conventional cultivation process of saffron.

**Claims**
**Claims for the following Contracting States : DE, GB**

1.  A process for culturing saffron stigma tissues which comprises the following steps:
    (a) picking the portion containing at least the upper, middle and lower parts (o, p and q) of the moiety continued from petal to ovary of saffron and cutting said portion into parts,
    (b) transplanting the cut parts on a liquid or solid LS medium or B5 medium, said medium containing at least one cytokinin selected from benzylaminopurine, kinetin and zeatin and at least one auxin selected from NAA, IBA and IAA as main hormones, and
    (c) subculturing the transplanted parts under stationary, rotary or shaking culture.

2.  A process according to claim 1, in which gibberellic acid (GA$_3$) is added as a further hormone.

3.  A process according to claim 1 or 2, in which the concentration of the cytokinin is $10^{-6} - 3 \times 10^{-5}$ and the concentration of the auxin $10^{-6} - 5 \times 10^{-5}$ (mol/l).

4.  A process according to any one of claims 1 to 3, in which the auxin is NAA.

5.  A process according to any one of claims 1 to 4, further including the step of:
    (d) transplanting the parts cultured on the LS medium or B5 medium to the B5 or LS medium respectively, followed by subculturing.

6.  A process according to claim 5, which the concentration of the main hormones in the medium for successive transplantation is lower than in the previous medium.

7.  A process according to claim 5 or 6, wherein transplanting and subculturing steps (b) and (c) are repeated successively at intervals of 5 − 8 weeks.

8.  A process according to any preceding claim, wherein said portion of saffron also contains at least the upper part of the ovary (h), the stigma style (d), and the upper, middle and lower parts of the style (e, f and g).

EP 0 261 862 B1

**Claims for the following Contracting States : ES, FR, IT**

1. A process for culturing saffron stigma tissues which comprises the following steps:
   (a) picking the portion containing at least the upper, middle and lower parts (o, p and q) of the moiety continued from petal to ovary of saffron and cutting said portion into parts,
   (b) transplanting the cut parts on a liquid or solid LS medium or B5 medium, said medium containing at least one cytokinin selected from benzylaminopurine, kinetin and zeating and at least one auxin selected from NAA, IBA, IAA as main hormones,
   (c) subculturing the transplanted parts under stationary, rotary or shaking culture, and
   (d) transplanting the parts subcultured on the LS medium or B5 medium to the B5 or LS medium respectively, followed by subculturing, and in which the concentration of the main hormones in the medium for successive transplantation is lower than in the previous medium.

2. A process according to claim 1, in which gibberellic acid ($GA_3$) is added as a further hormone.

3. A process according to claim 1 or 2, in which the concentration of the cytokinin is $10^{-6}$ – $3x10^{-5}$ and the concentration of the auxin $10^{-6}$ – $5x10^{-5}$ (mol/l).

4. A process according to any one of claims 1 to 3, in which the auxin is NAA.

5. A process according to any preceding claim, wherein the transplanting and sub–culturing steps (b) and (c) are repeated successively at intervals of 5–8 weeks.

6. A process according to any preceding claim, wherein said portion of saffron also contains at least the upper part of the ovary (h), the stigma style (d), and the upper, middle and lower parts of the style (e, f and g).

**Patentansprüche**
**Patentansprüche für folgende Vertragsstaaten : DE, GB**

1. Verfahren zum Züchten von Safranstigmageweben, umfassend die Schritte:
   (a) Pflücken des Abschnitts, der mindestens die oberen, mittleren und unteren Teile (o, p und q) der Komponente enthält, die vom Blütenblatt bis zu den Fruchtknoten des Safrans verläuft, und Schneiden des Abschnitts zu Stücken,
   (b) Transplantieren der geschnittenen Teile auf ein flüssiges oder festes LS– oder B5–Medium, wobei das Medium mindestens ein Cytokinin enthält, das ausgewählt wird aus Benzylaminopurin, Kinetin und Zeatin, und mindestens ein aus NAA, IBA und IAA als Haupthormone ausgewähltes Auxin, und
   (c) In–Zweitkulturnehmen der transplantierten Teile unter stationärer, rotierender oder schüttelnder Kultur.

2. Verfahren nach Anspruch 1, bei welchem Gibberellinsäure ($GA_3$) als ein weiteres Hormon zugesetzt wird.

3. Verfahren nach Anspruch 1 oder 2, bei welchem die Konzentration des Cytokinins $10^{-6}$ – $3x10^{-5}$ und die Konzentration des Auxin $10^{-6}$ – $5x10^{-5}$ (mol/l) betragen.

4. Verfahren nach einem der Ansprüche 1 bis 3, bei welchem das Auxin NAA ist.

5. Verfahren nach einem der Ansprüche 1 bis 4, ferner umfassend den Schritt :
   (d) Transplantieren der auf dem LS–Medium oder B5–Medium gezüchteten Teile auf das B5– bzw. LS–Medium und nachfolgendem In–Zweitkulturnehmen.

6. Verfahren nach Anspruch 5, bei welchem die Konzentration der Haupthormone in dem Medium für die aufeinanderfolgende Transplantation kleiner ist als in dem vorangegangenen Medium.

7. Verfahren nach Anspruch 5 oder 6, bei welchem die Schritte (b) und (c) des Transplantierens und In–Zweitkulturnehmens in Abständen von 5 bis 8 Wochen wiederholt werden.

14

**8.** Verfahren nach einem der vorstehenden Ansprüche, bei welchem der Abschnitt des Safrans ebenfalls mindestens den oberen Teil des Fruchtknotens (h), den Stigmagriffel (d) und die oberen, mittleren und unteren Teile des Griffels (e, f und g) enthält.

**Patentansprüche für folgende Vertragsstaaten : ES, FR, IT**

**1.** Verfahren zum Züchten von Safranstigmageweben, umfassend die Schritte:
(a) Pflücken des Abschnitts, der mindestens die oberen, mittleren und unteren Teile (o, p und q) der Komponente enthält, die vom Blütenblatt bis zu den Fruchtknoten des Safrans verläuft, und Schneiden des Abschnitts zu Stücken,
(b) Transplantieren der geschnittenen Teile auf ein flüssiges oder festes LS − oder B5 − Medium, wobei das Medium mindestens ein Cytokinin enthält, das ausgewählt wird aus Benzylaminopurin, Kinetin und Zeatin, und mindestens ein aus NAA, IBA, IAA als Haupthormone ausgewähltes Auxin,
(c) In − Zweitkulturnehmen der transplantierten Teile unter stationärer, rotierender oder schüttelnder Kultur.
(d) Transplantieren der auf dem LS − Medium oder B5 − Medium gezüchteten Teile auf das B5 − bzw. LS − Medium, nachfolgendes In − Zweitkulturnehmen, und in welchem die Konzentration der Haupthormone in dem Medium für die aufeinanderfolgende Transplantation kleiner ist als in dem vorangegangenen Medium.

**2.** Verfahren nach Anspruch 1, bei welchem Gibberellinsäure (GA$_3$) als ein weiteres Hormon zugesetzt wird.

**3.** Verfahren nach Anspruch 1 oder 2, bei welchem die Konzentration des Cytokinins $10^{-6}$ − $3x10^{-5}$ und die Konzentration des Auxin $10^{-6}$ − $5x10^{-5}$ (mol/l) betragen.

**4.** Verfahren nach einem der Ansprüche 1 bis 3, bei welchem das Auxin NAA ist.

**5.** Verfahren nach einem der vorstehenden Ansprüche, bei welchem die Schritte (b) und (c) des Transplantierens und In − Zweitkulturnehmens in Abständen von 5 bis 8 Wochen wiederholt werden.

**6.** Verfahren nach einem der vorstehenden Ansprüche, bei welchem der Abschnitt des Safrans ebenfalls mindestens den oberen Teil des Fruchtknotens (h), den Stigmagriffel (d) und die oberen, mittleren und unteren Teile des Griffels (e, f und g) enthält.

**Revendications**
**Revendications pour les Etats contractants suivants : DE, GB**

**1.** Procédé de culture de tissus de stigmates de safran qui comprend les étapes suivantes:
(a) prélèvement de la portion contenant au moins les fractions supérieure, médiane et inférieure (o, p et q) de la partie continue qui va des pétales à l'ovaire du safran et découpe de ladite portion en morceaux
(b) transplantation des morceaux découpés sur un milieu B5 ou un milieu LS liquide ou solide, ledit milieu contenant au moins une cytoquinine choisie parmi la benzylaminopurine, la cinétine et la zéatine et au moins une auxine choisie parmi la NAA, l'IBA et l'IAA comme hormones principales et
(c) sous − culture des morceaux transplantés en culture stationnaire, rotative ou agitée.

**2.** Procédé selon la revendication 1, dans lequel on ajoute de l'acide gibbérellique (GA$_3$) comme hormone additionnelle.

**3.** Procédé selon la revendication 1 ou 2, dans lequel la concentration de la cytoquinine et de $10^{-6}$ à $3 \times 10^{-5}$ et la concentration de l'auxine est de $10^{-6}$ à $5 \times 10^{-5}$ (mole/ℓ).

**4.** Procédé selon l'une quelconque des revendications 1 à 3, dans lequel l'auxine est la NAA.

**5.** Procédé suivant l'une quelconque des revendications 1 à 4, comprenant en outre l'inclusion de l'étape constituée par (d) la transplantation des morceaux cultivés sur le milieu LS ou sur le milieu B5 respectivement sur le milieu B5 ou Ls, suivie d'une sous – culture.

**6.** Procédé selon la revendication 5, dans lequel la concentration en hormone principale dans le milieu pour transplantation suivante est inférieure à celle du milieu précédent.

**7.** Procédé selon la revendication 5 ou 6, dans lequel les étapes de transplantation et de sous – culture (b) et (c) sont répétées successivement à des intervalles de 5 à 8 semaines.

**8.** Procédé selon l'une quelconque des revendications précédentes, dans lequel ladite portion de safran contient aussi au moins la partie supérieure de l'ovaire (h), le style du stigmate (d) et les fractions supérieure, médiane et inférieure du style (e, f et g).

**Revendications pour les Etats contractants suivants : ES, FR, IT**

**1.** Procédé de culture de tissus de stigmates de safran qui comprend les étapes suivantes:
(a) prélèvement de la portion contenant au moins les fractions supérieure, médiane et inférieure (o, p et q) de la partie continue qui va des pétales à l'ovaire du safran et découpe de ladite portion en morceaux
(b) transplantation des morceaux découpés sur un milieu B5 ou un milieu LS liquide ou solide, ledit milieu contenant au moins une cytoquinine choisie parmi la benzylaminopurine, la cinétine et la zéatine et au moins une auxine choisie parmi la NAA, l'IBA et l'IAA comme hormones principales et
(c) sous – culture des morceaux transplantés en culture stationnaire, rotative ou agitée et
(d) transplantation des fractions mises en sous – culture sur le milieu LS ou le milieu B5 respective – ment sur le milieu B5 ou LS, suivie de la sous – culture et dans lequel la concentration des hormones principales dans le milieu pour transplantation suivante est inférieure à celle du milieu précédent.

**2.** Procédé selon la revendication 1, dans lequel on ajoute de l'acide gibbérellique ($GA_3$) comme hormone additionnelle.

**3.** Procédé selon la revendication 1 ou 2, dans lequel la concentration de la cytoquinine et de $10^{-6}$ à 3 x $10^{-5}$ et la concentration de l'auxine est de $10^{-6}$ à 5 x $10^{-5}$ (mole/$\ell$).

**4.** Procédé selon l'une quelconque des revendications 1 à 3, dans lequel l'auxine est la NAA.

**5.** Procédé suivant l'une quelconque des revendications précédentes, dans lequel les étapes de trans – plantation et de sous – culture (b) et (c) sont répétées successivement à des intervalles de 5 à 8 semaines.

**6.** Procédé sel on l'une quelconque des revendications précédentes, dans lequel ladite portion de safran contient aussi au moins la partie supérieure de l'ovaire (h), le style du stigmate (d) et les fractions supérieure, médiane et inférieure du style (e, f et g).

# FIG. 1

Fig. 2

Fig. 3